# EUROPÄISCHE PATENTSCHRIFT

(11) **EP 2 475 333 B1**
(45) Veröffentlichungstag und Bekanntmachung des Hinweises auf die Patenterteilung: **28.12.2016**
(21) Anmeldenummer: 10754319.1
(22) Anmeldetag: 10.09.2010
(51) Int. Cl.: A61F 2/38, A61F 2/30

(54) **KNIEGELENKENDOPROTHESE**
KNEE JOINT ENDOPROSTHESIS
ENDOPROTHÈSE DE L'ARTICULATION DU GENOU

(30) Priorität: 10.09.2009 DE 102009029360
(43) Veröffentlichungstag der Anmeldung: 18.07.2012
(73) Patentinhaber: Aesculap AG, 78532 Tuttlingen (DE)
(72) Erfinder: HAGEN, Thomas, 78532 Tuttlingen (DE); BLÖMER, Wilhelm, 88690 Unteruhldingen-Mühlhofen (DE); JANSSON, Volkmar, 82205 Gilching (DE)
(74) Vertreter: Hoeger, Stellrecht & Partner Patentanwälte mbB
(86) Internationale Anmeldenummer: PCT/EP2010/063325
(87) Internationale Veröffentlichungsnummer: WO 2011/029908

(56) Entgegenhaltungen:
- EP-A1- 2 464 314
- EP-A2- 1 378 216
- WO-A1-98/46171
- WO-A1-2011/018441
- DE-U1-202008 004 709
- FR-A1- 2 707 871

## Beschreibung

Die vorliegende Erfindung betrifft eine Kniegelenkendoprothese mit einem Femurteil und einem relativ zu und an diesem beweglichen gelagerten Meniskusteil, welches Femurteil eine mediale und eine laterale Kondyle umfasst, welche eine mediale und eine laterale Kondylenfläche aufweisen, welches Meniskusteil eine mediale und eine laterale Gelenkfläche aufweist, an welchen die medialen und lateralen Kondylenflächen mindestens teilweise anliegen, wobei die mediale Kondyle und die mediale Gelenkfläche geformt sind zur Ausbildung eines Rotationsgelenks mit einem Rotationsgelenkzentrum, wobei eine Abrollbewegungsführungseinrichtung vorgesehen ist zum definierten Abrollen der lateralen Kondylenfläche und der lateralen Gelenkfläche aneinander längs einer in Abhängigkeit eines Flexionswinkels zwischen Femurteil und Meniskusteil definierten, um das Rotationsgelenkzentrum verlaufenden, gekrümmten Bahn.

Kniegelenkendoprothesen mit einem Femurteil und einem relativ zu und an diesem beweglichen gelagerten Meniskusteil, welches Femurteil eine mediale und eine laterale Kondyle umfasst, welche eine mediale und eine laterale Kondylenfläche aufweisen, welches Meniskusteil eine mediale und eine laterale Gelenkfläche aufweist, an welchen die medialen und lateralen Kondylenflächen mindestens teilweise anliegen, wobei die mediale Kondyle und die mediale Gelenkfläche geformt sind zur Ausbildung eines Rotationsgelenks mit einem Rotationsgelenkzentrum, orientieren sich in ihrem Aufbau an einem gesunden Kniegelenk. Ein solches bewegt sich während der Kniebeugung, auch als Flexion bezeichnet, auf der lateralen Seite mehr nach posterior als auf der medialen Seite und ermöglicht durch die damit erzeugte axiale Rotation während der Beugung des Kniegelenks eine optimale Führung der Patella durch die Trochlea. Die Lage der Trochlea zur Patella hat einen sehr starken Einfluss auf eine optimale Kraftumlenkung, insbesondere auf das Streckvermögen. Daher ist es bekannt, Kniegelenkendoprothesen der eingangs beschriebenen Art in Form nicht gekoppelter, bikondylärer Knieimplantate mit einer - an der Tibia oder an einem an der Tibia festgelegten Tibiateil - fixierten Meniskuskomponente auszubilden, welche eine ungezwungene axiale Rotationsmöglichkeit der Tibia zum Femur ermöglichen.

Bei einer schlechten Bandsituation oder bei teilweise fehlenden Kreuzbändern wird durch die bekannte Implantatkonstruktion eine posteriore Verschiebung der Femurkomponente erzwungen, zumindest auf der lateralen Seite. Auf diese Weise kommt der Femur bei einer Flexion später in Kontakt zum Meniskusteil, das insbesondere aus Polyethylen (PE) hergestellt ist, beziehungsweise mit der Tibiakomponente, so dass sich theoretisch auf diese Weise ein größerer Beugungs- oder Flexionswinkel zwischen Tibia und Femur beziehungsweise deren Längsachsen realisieren lässt. Die posteriore Translation wird üblicherweise durch das Zusammenwirken eines Vorsprungs ("post") und eines entsprechenden Nockens ("cam") zwischen dem Femurteil und dem Meniskusteil oder über eine dritte Kondyle realisiert. Allerdings kann dadurch eine perfekte Lageausrichtung der Trochlea, wie dies beim natürlichen, gesunden Kniegelenk der Fall ist, nicht realisiert werden.

Aus der EP 1 378 216 A2 ist eine Kniegelenkendoprothese mit einem um einen am Tibiateil fixierten Bolzen rotierbaren Meniskusteil bekannt. In der WO 98/ 46171 A1 ist eine Kniegelenkendoprothese mit Führungsflächen zur Steuerung einer Bewegung in anterior-posteriorer Richtung offenbart. Eine weitere Kniegelenkendoprothese ist in der DE 20 2008 004 709 U1 beschrieben. Und schließlich ist aus der FR 2 707 871 A1 eine Kniegelenkendoprothese mit einem medialseitig rotierbar gelagertem Meniskusteil bekannt. In der EP 2 464 314 A1 sowie in der WO 2011/018441 A1 sind weitere Kniegelenkendoprothesen offenbart.

Es ist daher insbesondere eine Aufgabe der vorliegenden Erfindung, eine Kniegelenkendoprothese der eingangs beschriebenen Art so zu verbessern, dass eine Beugung des Kniegelenks verbessert wird, um eine optimale Führung der Patella durch die Trochlea zu ermöglichen und so die physiologische Kinematik des gesunden Kniegelenks zu erhalten.

Diese Aufgabe wird bei einer Kniegelenkendoprothese der eingangs beschriebenen Art erfindungsgemäß dadurch gelöst, dass das Rotationsgelenkzentrum in Form eines relativ zum Femurteil und zum Meniskusteil längs einer in Abhängigkeit eines Flexionswinkels zwischen Femurteil und Meniskusteil von anterior nach posterior verlaufenden Rotationsgelenkzentrumsbahn wandernden Gelenkzentrums ausgebildet ist.

Die erfindungsgemäß vorgeschlagene Kniegelenkendoprothese ermöglicht eine zwangsgeführte außermittige Rotation des durch die Kniegelenkendoprothese ausgebildeten künstlichen Kniegelenks bei einer Beugung desselben. Medialseitig ermöglicht die Kniegelenkendoprothese eine Rotation um das Rotationsgelenkzentrum, lateralseitig wird mittels der Abrollbewegungsführungseinrichtung während der Flexion des Kniegelenks, ausgehend zum Beispiel von der Streckung des Beines des Patienten, keine Gleitbewegung zwischen dem Femurteil und dem Meniskusteil zugelassen, sondern eine Abrollbewegung mit dem Effekt, dass sich dabei die laterale Kondyle des Femurteils etwas in posteriorer Richtung bewegt längs der um das Rotationsgelenkzentrum verlaufenden, gekrümmten Bahn. Die Rotation des Kniegelenks erfolgt somit automatisch zwangsgeführt und nicht durch noch vorhandene Weichteile initiiert. Mit der vorgeschlagenen Kniegelenkendoprothese lässt sich somit eine zwangsgeführte Rotation in der Beinachse beim Durchbewegen des Gelenks erzeugen und damit verbunden die Patella verbessert in der nun anatomisch ausgerichteten Trochlea führen. Weiterer Vorteil ist die Erhöhung der Kraftleistung der Beinmechanik bedingt durch die nun mögliche optimale Patellaführung, also eine verbesserte Quadrizepsleistung. Patella-Komplikationen, insbesondere eine Subluxation oder eine Luxation verbunden mit Schmerzen und Gelenkversagen, werden durch die verbesserte Patellaführung reduziert. Dadurch, dass auf die eingangs beschriebene Vorsprungs-/Nocken-Mechanik ("post/cam") zur Zwangsführung des Femur relativ zur Tibia nach posterior in Folge der Beugung verzichtet werden kann, weist das Meniskusteil eine deutlich höhere Lebensdauer auf. Ferner sind die Gefahr von Brüchen oder starkem Verschleiß des - nicht vorhandenen - Vorsprungs vollständig eliminiert. Des Weiteren können insgesamt eine Baugröße des Meniskusteils verringert und damit auch die OP-Technik vereinfacht werden. Zudem ist eine individuellere Versorgung des jeweiligen Patienten möglich. Gemäß der Erfindung ist vorgesehen sein, dass das Rotationsgelenkzentrum in Form eines relativ zum Femurteil und zum Meniskusteil längs einer in Abhängigkeit eines Flexionswinkels zwischen Femurteil und Meniskusteil von anterior nach posterior verlaufenden Rotationsgelenkzentrumsbahn wandernden Gelenkzentrums ausgebildet ist. Das wandernde Gelenkzentrum ermöglicht insbesondere in Folge einer Beugung des Kniegelenks eine Bewegung des Femurteils medialseitig etwas in posteriorer Richtung in Folge einer Beugung des Kniegelenks. Dabei kann es sich um eine translatorische Gleit- und/oder Abrollbewegung des Femurteils und des Meniskusteils aneinander handeln.

Günstig ist es, wenn das Rotationsgelenkzentrum in Form eines relativ zum Femurteil und zum Meniskusteil ortsfesten Gelenkzentrums ausgebildet ist. Ein derartiges ortsfestes Gelenkzentrum lässt sich auf einfache Weise ausbilden. Zudem lässt sich so auch das Kniegelenk bei einem sehr schwachen oder degenerierten Bandapparat besser stabilisieren.

Vorzugsweise ist das Rotationsgelenk in Form eines Kugelgelenks ausgebildet. Ein Kugelgelenk ermöglicht auf einfache Weise insbesondere die Ausbildung eines ortsfesten Gelenkzentrums.

Der Aufbau der Kniegelenkendoprothese wird besonders einfach, wenn die mediale Kondylenfläche einen kugeligen Kondylenflächenbereich und wenn die mediale Gelenkfläche einen zur medialen Kondylenfläche korrespondierenden hohlkugeligen Gelenkflächenbereich umfasst. Der kugelige Kondylenflächenbereich kann somit direkt im oder am hohlkugeligen Gelenkflächenbereich gelagert werden, wobei deren Krümmungsradien vorzugsweise derart aneinander angepasst sind, dass ein relativ zum Femurteil und zum Meniskusteil ortsfestes Gelenkzentrum definiert wird.

Vorteilhaft ist es, wenn das ortsfeste Gelenkzentrum derart ausgebildet ist, dass es ausschließlich eine Gleitbewegung des Femurteils und des Meniskusteils relativ zueinander ermöglicht. Auf diese Weise wird verhindert, dass sich das Femurteil relativ zum Meniskusteil in Folge einer Beugung des Kniegelenks in posteriorer Richtung bewegen kann.

Vorteilhafterweise ist die Kniegelenkendoprothese derart ausgebildet, dass die Rotationsgelenkzentrumsbahn geradlinig verläuft oder von der Meniskuskomponente in medialer Richtung weg weisend konkav gekrümmt ist. Durch entsprechende Ausgestaltung der medialen Kondylenfläche und der medialen Gelenkfläche lässt sich die Rotationsgelenkzentrumsbahn vorgeben, beispielsweise durch entsprechend gekrümmte mediale Kondylenflächenbereiche und einen korrespondierenden medialen Gelenkflächenbereich des Meniskusteils.

Auf besonders einfache Weise lässt sich ein wanderndes Gelenkzentrum ausbilden, wenn ein Krümmungsradius der medialen Gelenkfläche größer ist als ein Krümmungsradius der medialen Kondylenfläche. Es handelt sich dabei vorzugsweise um diejenigen Flächenbereiche, längs denen das Femurteil und das Meniskusteil aneinander anliegen, wenn das Kniegelenk gebeugt wird, also bei einer Flexion oder einer entsprechenden Streckung, die auch als Extension oder Extensionsbewegung bezeichnet wird. Auf diese Weise wird eine Bewegung der medialen Kondyle des Femurteils relativ zur medialen Gelenkfläche des Meniskusteils bei einer Flexionsbewegung in posteriorer Richtung ermöglicht. Dabei sind das Meniskusteil und das Femurteil vorzugsweise derart ausgebildet, dass ausschließlich eine Gleitbewegung zwischen der medialen Kondyle und der medialen Gelenkfläche ermöglicht wird.

Günstig ist es, wenn das wandernde Gelenkzentrum derart ausgebildet ist, dass es eine überlagerte Gleit-/Abrollbewegung des Femurteils und des Meniskusteils relativ zueinander ermöglicht. Somit kann zumindest teilweise auch medialseitig eine definierte Translationsbewegung des Femurteils relativ zum Meniskusteil in Folge einer Flexionsbewegung des Knies erreicht werden.

Um auf einfache Weise eine Zwangsführung zur Vorgabe einer Abrollbewegung der lateralen Kondyle und der lateralen Gelenkfläche relativ zueinander zu ermöglichen, ist es vorteilhaft, wenn die Abrollbewegungsführungseinrichtung miteinander zusammenwirkende erste und zweite Führungselemente umfasst, welche am Femurteil und am Meniskusteil ausgebildet sind zum Definieren eines in Abhängigkeit eines Flexionswinkels zwischen Femurteil und Meniskusteil von anterior nach posterior und umgekehrt um das Rotationsgelenkzentrum wandernden Kontaktflächenbereichs. Durch die ersten und zweiten Führungselemente wird eine Gleitbewegung zwischen der lateralen Kondyle und der lateralen Gelenkfläche vollständig oder im Wesentlichen vollständig verhindert. Analog wie beim ineinander Greifen der Zähne eines Zahnrads und einer Zahnstange wird so eine Abrollbewegung des Kniegelenks lateralseitig durch die Führungselemente erzwungen. Die ersten und zweiten Führungselemente können jedoch auch derart ausgebildet sein, dass keine ausschließliche Abrollbewegung erzwungen wird, sondern auch noch, insbesondere in einem eingeschränkten Umfang, eine Gleitbewegung lateralseitig möglich ist.

Für eine besonders optimierte Führung der Patella in der Trochlea ist es vorteilhaft, wenn die Abrollbewegungsführungseinrichtung ausgebildet ist, um ausschließlich eine Abrollbewegung zwischen der lateralen Kondylenfläche und der lateralen Gelenkfläche zu ermöglichen. Wie bereits dargelegt kann dies insbesondere dadurch erreicht werden, dass die ersten und zweiten Führungselemente derart ineinander greifend ausgebildet sind, dass sie ähnlich wie bei dem ineinander Greifen von Zähnen eines Zahnrads und einer Zahnstange, keine Gleitmöglichkeit der Teile relativ zueinander gestatten.

Auf besonders einfache Weise lassen sich die Führungselemente ausbilden, wenn das Femurteil mindestens ein erstes und wenn das Meniskusteil mindestens ein zweites Führungselement aufweist, welche mindestens einen ersten und einen zweiten Führungselemente mindestens teilweise aneinander anliegende erste und zweite Führungselementeflächen definieren. Die aneinander anliegenden ersten und zweiten Führungselementflächen erzwingen in Folge einer Bewegung des Kniegelenks, also beispielsweise bei einer Beugung oder einer Streckung, eine lateralseitige Abrollbewegung des Femurteils und des Meniskusteils relativ zueinander.

Vorteilhaft ist es wenn, die erste Führungselementfläche mindestens einen in Richtung auf das Meniskusteil weisenden konkaven Flächenbereich aufweist. Dadurch kann beispielsweise ein entsprechender konvexer Flächenbereich des Meniskusteils in den konkaven Flächenbereich des Femurteils bei der gewünschten Abrollbewegung in Folge einer Flexion des Kniegelenks eingreifen.

Um eine möglichst gute Führung oder Verzahnung zwischen dem Femurteil und dem Meniskusteil zum Erzwingen einer lateralseitigen Abrollbewegung zu erreichen, ist es günstig, wenn die erste Führungselementfläche zwei oder mehr, durch jeweils einen konvexen Flächenbereich getrennte konkave Flächenbereiche aufweist. Diese können relativ zueinander in einer Reihe, die geradlinig oder gekrümmt geformt sein kann, hintereinander oder auch teilweise nebeneinander oder versetzt zueinander nebeneinander und hintereinander angeordnet sein.

Damit die ersten und zweiten Führungselementflächen auf einfacher Weise zusammenwirken können, ist es günstig, wenn die zweite Führungselementfläche mindestens einen in Richtung auf das Femurteil weisenden konvexen Flächenbereich aufweist. Der konvexe Flächenbereich kann insbesondere einen konkaven Flächenbereich des Femurteils aufnehmen, um eine Abrollbewegung des Femurteils und des Meniskusteils relativ zueinander zu erzwingen.

Um möglichst über einen großen Bereich eine definierte Abrollbewegung vorgeben zu können, ist es günstig, wenn die zweite Führungselementfläche zwei oder mehr, durch jeweils einen konkaven Flächenbereich getrennte konvexe Flächenbereiche aufweist.

Eine besonders geringe Baugröße des Meniskusteils lässt sich erreichen, wenn insbesondere die ersten und zweiten Führungselemente im Bereich der lateralen Gelenkfläche und der lateralen Kondylenfläche ausgebildet sind. Insbesondere können die ersten und zweiten Führungselemente in die laterale Gelenkfläche beziehungsweise in die laterale Kondylenfläche integriert und jeweils einstückig mit diesen ausgebildet sein. Damit kann eine Führung direkt in den Bereichen erreicht werden, wo der Femurteil und der Meniskusteil lateralseitig miteinander in Kontakt stehen.

Vorzugsweise weisen die ersten und zweiten Führungselemente erste und zweite Führungselementflächen auf, welche mindestens teilweise die laterale Gelenkfläche und die laterale Kondylenfläche bilden. Damit lassen sich das Femurteil und das Meniskusteil aneinander in den Bereichen führen, in denen sie miteinander in Kontakt stehen.

Vorteilhafterweise weist die laterale Kondylenfläche mindestens einen in Richtung auf das Meniskusteil weisenden konkaven Flächenbereich auf. Ein solcher konkaver Flächenbereich lässt sich beispielsweise in Form einer Vertiefung der ansonsten vorzugsweise überwiegend vom Femurteil weg weisend konvex gekrümmten Femurgelenkfläche ausbilden. In die Vertiefung oder Ausnehmung kann ein entsprechender, konvex vom Meniskusteil weg weisend abstehender Vorsprung oder eine Erhöhung eingreifen und so bei einer Flexion des Kniegelenks ausgehend von einer Streckstellung in eine beliebige Beugestellung eine erzwungene Abrollbewegung bewirken.

Gemäß einer weiteren bevorzugten Ausführungsform der Erfindung kann vorgesehen sein, dass die laterale Kondylenfläche zwei oder mehr, durch jeweils einen konvexen Flächenbereich getrennte konkave Flächenbereiche aufweist. So lässt sich auf einfache Weise eine zwei- oder mehrzähnige Verzahnung zwischen dem Femurteil und dem Meniskusteil ausbilden, um eine Abrollbewegung in Folge einer Flexionsbewegung des Kniegelenks zu erzwingen. Dabei ist anzumerken, dass die ineinander greifenden Vorsprünge und Ausnehmungen, die konvexe und konkave Flächenbereiche definieren, unterschiedlich geformt oder aber auch, wie bei einer Verbindung zwischen einem Zahnrad und einer Zahnstange, identisch geformt sein können.

Um das Zusammenwirken der ersten und zweiten Führungselemente weiter zu vereinfachen und zu optimieren, ist es günstig, wenn die laterale Gelenkfläche mindestens einen in Richtung auf das Femurteil weisenden konvexen Flächenbereich aufweist. Der konvexe Flächenbereich, der beispielsweise einen Teil einer äußeren Oberfläche eines Vorsprungs ausbilden kann, kann so auf einfache Weise in eine entsprechende Ausnehmung mit konkavem Flächenbereich des Femurteils eingreifen.

Ein optimiertes Zusammenwirken der ersten und zweiten Führungselemente ist insbesondere dann möglich, wenn die laterale Gelenkfläche zwei oder mehr, durch jeweils einen konkaven Flächenbereich getrennte konvexe Flächenbereiche aufweist.

Grundsätzlich wäre es denkbar, das Meniskusteil direkt an der Tibia festzulegen. Um gegebenenfalls das Austauschen des Meniskusteils in Folge von Verschleiß oder einer Beschädigung zu erleichtern, ist es vorteilhaft, wenn die Kniegelenkendoprothese ferner ein das Meniskusteil tragendes Tibiateil umfasst. Das Tibiateil kann so zunächst in einer Tibia eines Patienten verankert, beispielsweise durch Einzementieren oder Verschrauben, und anschließend mit dem Meniskusteil verbunden werden.

Bei einer Vielzahl von Kniegelenkendoprothesen ist vorgesehen, das Meniskusteil und das Tibiateil beweglich aneinander zu lagern. Gemäß einer bevorzugten Ausführungsform der vorliegenden Erfindung ist es jedoch günstig, wenn das Meniskusteil und das Tibiateil unbeweglich miteinander verbunden sind. Auf diese Weise kann das Meniskusteil in definierter Weise relativ zur Tibia des Patienten festgelegt werden. Die zur möglichst physiologischen Rekonstruktion eines Kniegelenks ausgebildeten Femur- und Meniskusteile ermöglichen in der oben beschriebenen Weise eine zumindest teilweise erzwungene Abrollbewegung aneinander lateralseitig in Folge einer Flexionsbewegung des Knies.

Besonders einfach und kompakt ausbilden lassen sich das Femurteil und auch das Meniskusteil, wenn das Femurteil außer der lateralen und der medialen Kondyle keine weitere Kondyle umfasst. Das Meniskusteil umfasst dann also ausschließlich eine laterale und eine mediale Kondyle. Des Weiteren ist bei der Kniegelenkendoprothese vorzugsweise kein Vorsprung in Form eines Pfostens ("post") oder dergleichen am Meniskusteil und/oder am Tibiateil vorgesehen, die zur Führung des Meniskusteils und des Femurteils während einer Beugungsbewegung des Knies zusammenwirken. Auf diese Weise lässt sich die Baugröße des Meniskusteils einfach und sicher minimieren.

Die Kniegelenkendoprothese lässt sich auf einfache Weise herstellen und zudem ihre Stabilität erhöhen, wenn das Femurteil und/oder das Meniskusteil und/oder das Tibiateil jeweils einstückig ausgebildet sind. Insbesondere können das Femurteil und das Tibiateil aus einem Implantatmaterial, beispielsweise einem Implantatstahl oder Titan, ausgebildet sein, das Meniskusteil aus einem abriebfesten Kunststoff, beispielsweise Polyethylen oder Polyethylen hoher Dichte und mit hohem Molekulargewicht.

Gemäß einer weiteren bevorzugten Ausführungsform der Erfindung kann es vorteilhaft sein, wenn das Femurteil und/oder das Tibiateil in Form modularer Prothesenteile ausgebildet sind. Sowohl das Femurteil als auch das Tibiateil lassen sich so individuell an die jeweilige Physiologie des Patienten anpassen. Beispielsweise können sowohl das Femurteil als auch das Tibiateil Schäfte aufweisen, die in entsprechende Kavitäten von Femur und Tibia eingesetzt und darin festgelegt werden können, beispielsweise mittels Knochenzement oder Knochenschrauben. Die Schäfte können ferner lösbar verbindbar mit weiteren Bestandteilen des Femurteils beziehungsweise des Tibiateils verbindbar sein, so dass sich bei Bedarf das Femurteil und das Tibiateil individuell von einem Operateur noch während eines chirurgischen Eingriffs zusammenstellen lassen, um eine bestmögliche Anpassung der Kniegelenkendoprothese an die Physiologie des Patienten zu ermöglichen.

Grundsätzlich wäre es zwar denkbar, das Femurteil und das Meniskusteil aus identischen Materialien herzustellen. Um eine Reibung und dadurch den Verschleiß durch Abrieb zu minimieren, ist es günstig wenn das Femurteil und das Meniskusteil aus unterschiedlichen Materialien hergestellt sind. Günstigerweise sind das Femurteil und das Tibiateil aus identischen Materialien hergestellt. Vorteilhaft ist es dabei, wenn das Femurteil aus einem abriebfesteren Material hergestellt ist als das Meniskusteil. Auf diese Weise lässt sich ein Verschleiß der Kniegelenkendoprothese auf den Teil verlagern, der gegebenenfalls einfacher ausgetauscht werden kann.

Die nachfolgende Beschreibung bevorzugter Ausführungsformen der Erfindung dient im Zusammenhang mit der Zeichnung der näheren Erläuterung. Es zeigen:
- Figur 1:: eine anteriore Draufsicht auf eine implantierte Kniegelenkendoprothese bei durchgestrecktem Kniegelenk;
- Figur 2:: eine anteriore perspektivische Explosionsdarstellung des Femurteils und des Meniskusteils der Kniegelenkendoprothese aus Figur 1 von oben;

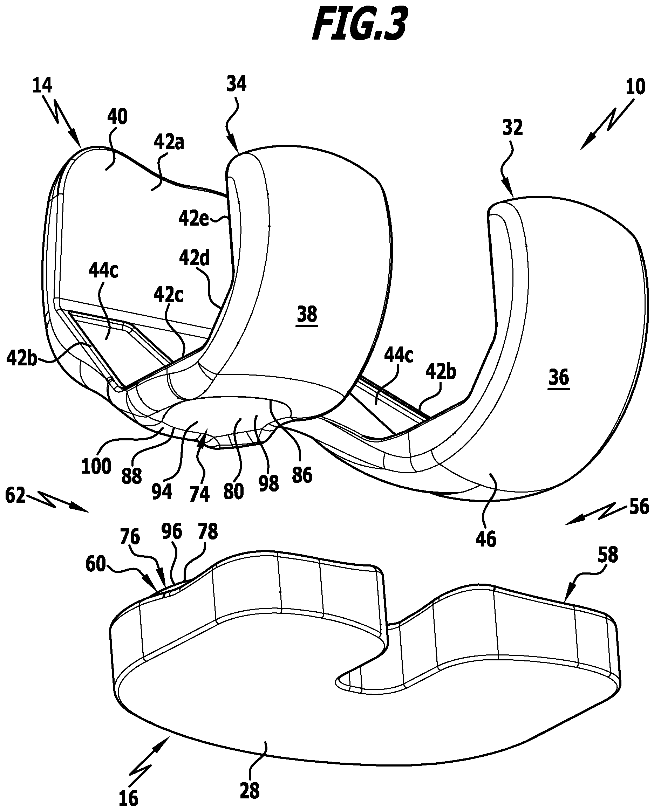

- Figur 3:: eine posteriore perspektivische Ansicht analog Figur 2 von unten;

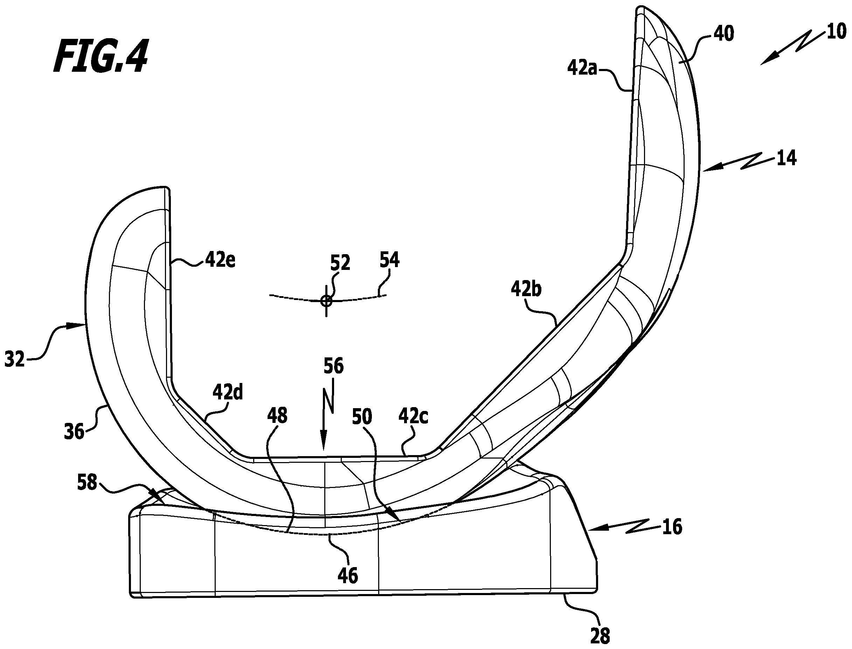

- Figur 4:: eine mediale Seitenansicht der beiden in den Figuren 2 und 3 dargestellten Teile der Kniegelenkendoprothese;

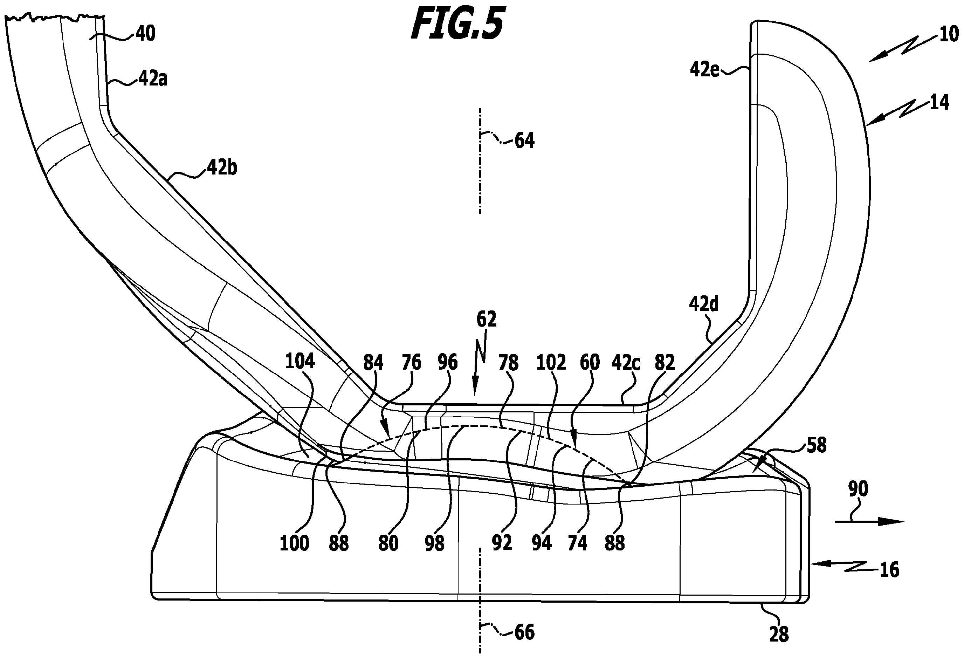

- Figur 5:: eine laterale Ansicht analog 4;

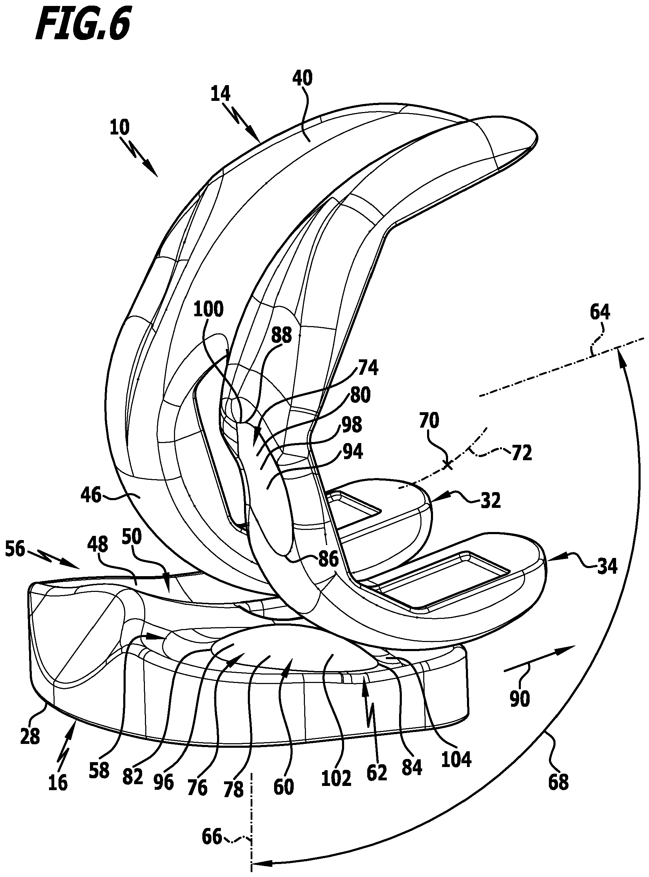

- Figur 6:: eine perspektivische Ansicht des Femurteils und des Meniskusteils in einer Flexionsstellung von etwa 90°; und

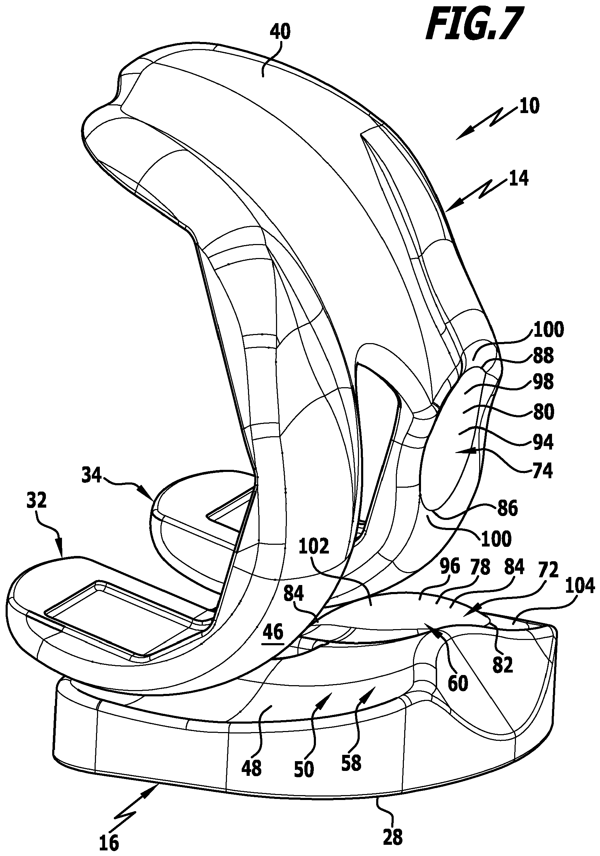

- Figur 7:: eine anterior-laterale Ansicht analog Figur 6.

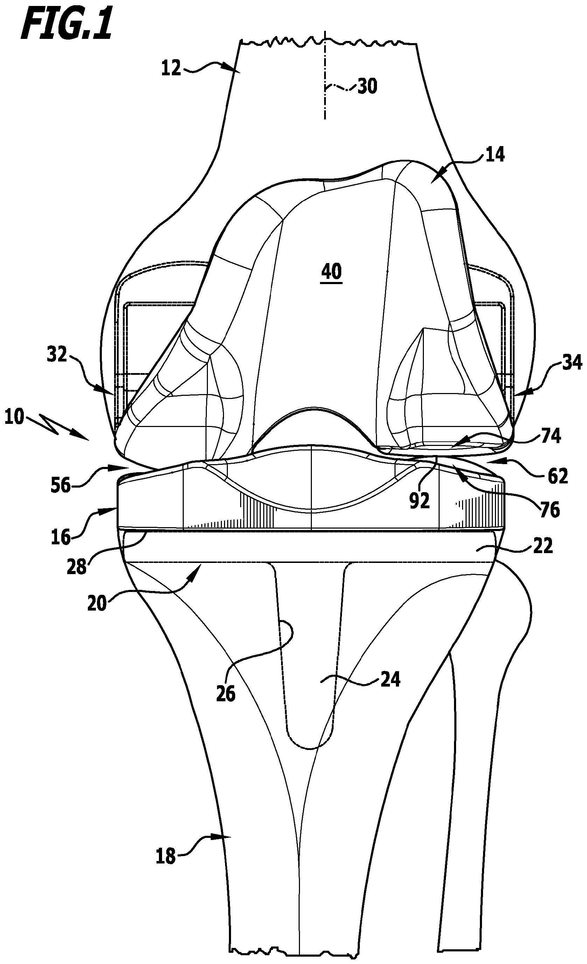

In Figur 1 ist eine insgesamt mit dem Bezugzeichen 10 bezeichnete Kniegelenkendoprothese dargestellt. Sie umfasst ein an einem Femur 12 festlegbares, bikondyläres Femurteil 14 sowie ein relativ zu und an diesem beweglich gelagertes Meniskusteil 16.

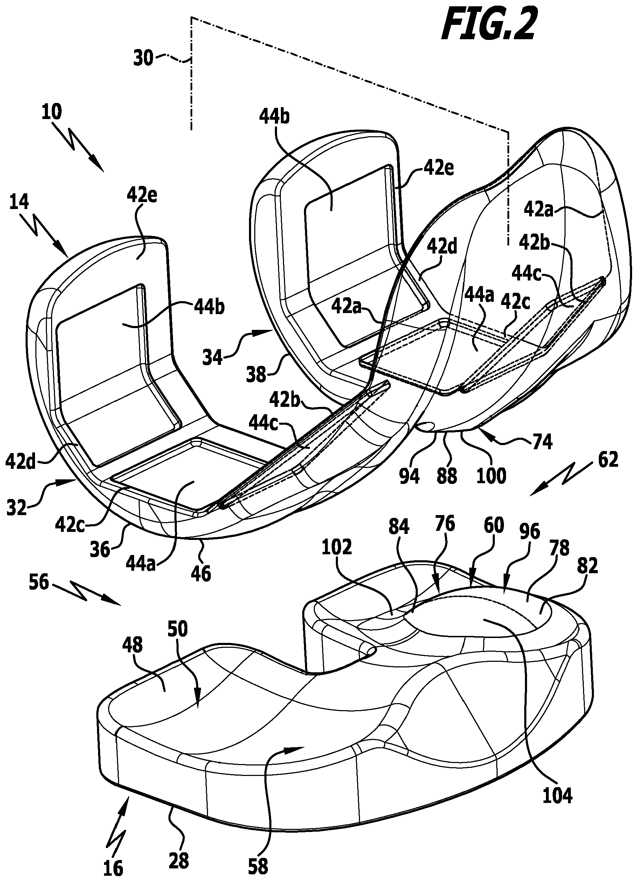

Das Meniskusteil 16 kann wahlweise direkt mit einer teilweise resizierten Tibia 18 eines Patienten verbunden werden, beispielsweise durch Schrauben oder Zementieren, oder alternativ an einem in Figur 2 gestrichelt dargestellten Tibiateil 20 der Kniegelenkendoprothese 10 unbeweglich festgelegt sein.

Das Tibiateil 20 umfasst eine Platte 22 sowie einen im Wesentlichen quer von dieser abstehenden Schaft 24, der in einer entsprechend geformten Kavität 26 der Tibia 18 festgelegt ist, beispielsweise mittels Knochenzement oder nicht dargestellten Knochenschrauben. Das Meniskusteil 16 ist mittels einer nicht dargestellten Verbindungsvorrichtung unbeweglich am Tibiateil 20 festgelegt. Die Verbindungsvorrichtung kann insbesondere in Form einer Rast- oder Schnappverbindung mit ineinander greifenden Rast- beziehungsweise Schnappelementen ausgebildet sein, die an der Platte 22 und an einer Unterseite 28, die ansonsten eben ausgebildet ist, ausgebildet sein. Alternativ kann die Verbindungsvorrichtung auch Befestigungselemente umfassen, beispielsweise Schrauben, mit denen das Meniskusteil unbeweglich am Tibiateil 20 festgelegt werden kann.

Das Femurteil 14, ebenso wie das Meniskusteil 16, ist bezogen auf eine Sagittalebene 30 unsymmetrisch ausgebildet und umfasst zwei Kondylen, nämlich eine mediale Kondyle 32 und eine laterale Kondyle 34, welche eine mediale Kondylenfläche 36 und eine laterale Kondylenfläche 38 aufweisen. Die Kondylenflächen 36 und 38 weisen vom Femurteil 14 weg in Richtung auf das Meniskusteil 16 hin.

Die Kondylen 32 und 34 sind in lateral-medialer Richtung voneinander beabstandet und an ihren anterioren Enden über ein Verbindungselement 40 einstückig miteinander verbunden. Vom Meniskusteil 16 weg weisende Anlageflächen 42a, 42b, 42c, 42d und 42e zum Anlegen des Meniskusteils an einen entsprechend präparierten Femur 12 sind relativ zueinander jeweils um 45° geneigt. In den Anlageflächen 42b und 42c ist eine erste Ausnehmung 44a ausgebildet, die in jeder der Anlageflächen 42b und 42c eine konstante Tiefe aufweist, sich aber nicht über die gesamte, von den beiden Anlagenflächen 42b und 42c definierte Fläche erstreckt. Eine weitere Ausnehmung 44b ist ausgehend von den Anlageflächen 42d und 42e in den Kondylen 32 und 34 ausgebildet. Die Anlageflächen 42a bis 42e in Verbindung mit den Ausnehmungen 44a und 44b der beiden Kondylen 32 und 34 dienen dazu, das Femurteil 14 an einem zuvor entsprechend einer von den Anlageflächen 42a bis 42e vorgegebenen Innenkontur des Femurteils 14 teilresizierten Femur festzulegen, vorzugsweise mit Knochenzement.

Die Kondylenflächen 36 und 38 sind unterschiedlich geformt. Die mediale Kondylenfläche 36 weist einen kugeligen Kondylenflächenbereich 46 auf, welcher vom Femurteil 14 weg weisend und ausschließlich konvex gekrümmt ist. Ein Krümmungsradius des kugeligen Kondylenflächenbereichs 46 ist etwas kleiner als ein hohlkugeliger Gelenkflächenbereich 48 einer medialen Gelenkfläche 50 des Meniskusteils 16, an welchem die mediale Kondylenfläche 36 mit dem kugeligen Kondylenflächenbereich 46 teilweise anliegt. Optional können die Krümmungsradien des kugeligen Kondylenflächenbereichs 46 und des hohlkugeligen Gelenkflächenbereichs 48 auch identisch ausgebildet sein, so dass ein Rotationsgelenkzentrum 52 eines Kugelgelenks definiert wird. Das Rotationsgelenkzentrum 52 kann in Form eines ortsfesten Gelenkzentrums ausgebildet sein, wenn der Radius des kugeligen Kondylenflächenbereichs 46 und des hohlkugeligen Gelenkflächenbereichs 48 identisch sind. Ist der wannenförmige, hohlkugelige Gelenkflächenbereich 48 weniger stark gekrümmt als der kugelige Kondylenflächenbereich 46, kann das Rotationsgelenkzentrum 52 längs einer Rotationsgelenkzentrumsbahn 54 von einer maximalen anterioren Position bei durchgestrecktem Kniegelenk in posteriorer Richtung hin zu einer maximalen posterioren Endposition wandern, in welcher das Kniegelenk maximal abgebeugt ist. Insgesamt bildet die mediale Kondyle 32 mit der medialen Gelenkfläche dann auch ein Rotationsgelenk 56 aus.

Die mediale Gelenkfläche 50 bildet einen Teil einer Oberseite 58 des Meniskusteils 16, welche ferner eine laterale Gelenkfläche 60 umfasst, welche zum Zusammenwirken mit der lateralen Kondylenfläche 38 in der nachfolgend beschriebenen Weise ausgebildet ist.

Wie eingangs erwähnt, sind Kniegelenkendoprothesen mit einem außermittigen Rotationsgelenk 52 wie oben beschrieben bekannt. Um in Folge einer Beugung des Kniegelenks ein definiertes Abrollen, vorzugsweise nur der lateralen Kondyle 34 und der lateralen Gelenkfläche 60, zu erreichen, umfasst die Kniegelenkendoprothese 10 eine Abrollbewegungsführungseinrichtung 62. Sie dient zum Erzwingen einer definierten Abrollbewegung der lateralen Kondylenfläche 38 und der lateralen Gelenkfläche 60 aneinander längs einer in Abhängigkeit eines Flexionswinkels 68 des Kniegelenks, also zwischen einer Längsachse 64 des Femurs und einer Längsachse 66 der Tibia, definierten, um das Rotationsgelenkzentrum 52 verlaufenden gekrümmten Bahn 72. Dies hat zur Folge, dass ein laterales Gelenkzentrum 70 in Folge der Beugung längs einer Bahn 72 in posteriorer Richtung wandert. Handelt es sich beim Gelenkzentrum 52 um ein ortsfestes Gelenkzentrum, dann verläuft die Bahn 72 um das ortsfeste Gelenkzentrum 52. Dies ergibt sich daraus, dass das Rotationsgelenk 56 in diesem Fall ein Kugelgelenk ist.

Die erzwungene Abrollbewegung wird durch die besondere Ausgestaltung der Abrollbewegungsführungseinrichtung 62 erreicht, die im Prinzip im Aufbau einem Zahnrad und einer Zahnstange entspricht, die relativ zueinander bewegt werden. Erste Führungselemente 74 entsprechen dabei Zähnen eines Zahnrads und zweite Führungselemente 76 am Meniskusteil 16 Zähnen der Zahnstange. Die ersten und zweiten Führungselemente 74 und 76 sind in Form von Vertiefungen 78 und 80 sowie Erhöhungen 82 und 84 am Meniskusteil 16 sowie 86 und 88 am Femurteil 14 ausgebildet. Sie sind derart angeordnet, dass die Erhöhungen 82, 84, 86 und 88 beim Beugen des Knies in die entsprechend geformten Vertiefungen 78 und 80 eintauchen. Durch die besondere Ausgestaltung der Vertiefungen 78 und 80 sowie der Erhöhungen 82, 84, 86 und 88 wird im Ergebnis eine Verzahnung erreicht, die eine Gleitbewegung der Kniegelenkendoprothese 10 lateralseitig, also zwischen der lateralen Kondylenfläche 38 und der lateralen Gelenkfläche 60 verhindert. Die ersten und zweiten Führungselemente 74 und 76 sind zudem vorzugsweise konzentrisch zum Rotationsgelenkzentrum 52 ausgebildet, so dass medialseitig eine Rotation um das Rotationsgelenkzentrum 52 und lateralseitig eine überlagerte Rotations-/Abrollbewegung um das Rotationsgelenkzentrum 52 erzwungen. Dabei wandert das Gelenkzentrum 70, welches im Wesentlichen durch die laterale Kondyle 34 definiert wird, längs der Bahn 72 mit zunehmendem Flexionswinkel 68 in Richtung des Pfeils 90 in posteriorer Richtung.

Bei einem wandernden Rotationsgelenkzentrum 52 verläuft die Rotationsgelenkzentrumsbahn 54 je nach Ausgestaltung der medialen Gelenkfläche 50 sowie der zugeordneten medialen Kondylenfläche 36 entweder geradlinig oder vom Meniskusteil 16 in medialer Richtung weg weisend konvex gekrümmt.

In analoger Weise wandert auch ein Kontaktflächenbereich 92 in posteriorer Richtung, wobei der Kontaktflächenbereich 92 definiert wird durch die aneinander anliegende laterale Kondylenfläche 38 und laterale Gelenkfläche 60.

Zur Führung des Femurteils 14 und des Meniskusteils 16 relativ zueinander definieren die ersten und zweiten Führungselemente 72 und 74 erste Führungselementflächen 94 und zweite Führungselementflächen 96. Die erste Führungselementfläche 94 weist mindestens einen konkaven Flächenbereich 98 auf, die von konvexen Flächenbereichen 100 seitlich begrenzt sind. In analoger Weise weist die zweite Führungselementfläche 96 mindestens einen, vorzugsweise zwei in Richtung auf das Femurteil 14 weisende konvexe Flächenbereiche 102 auf, die vorzugsweise durch einen konkaven Flächenbereich 104 voneinander getrennt sind.

Die ersten und zweiten Führungselemente 74, 76 sind im Bereich der lateralen Gelenkfläche 60 und der lateralen Kondylenfläche 38 ausgebildet, wodurch sich eine besonders kleine Baugröße des Meniskusteils 16 ergibt. Sie bilden somit mindestens teilweise die laterale Gelenkfläche 60 und die laterale Kondylenfläche 38.

Das Femurteil 14 und das Tibiateil 20 können optional in Form von modularen Prothesenteilen ausgebildet sein, welche aus mehreren, miteinander verbundenen und gegebenenfalls austauschbaren Teilen zusammengesetzt sein können.

Das beschriebene Implantatdesign, welches beim Bewegen des Beins eine zwangsgeführte axiale Rotation um das außermittige Rotationsgelenkzentrum 52, optional auch medialseitig überlagert mit einer posterioren Translation, erzeugt und damit eine definierte Ausrichtung der Trochlea ermöglicht, trägt wesentlich dazu bei, die physiologische Kinematik des Kniegelenks zu erhalten. Hierfür sind weder weitere Führungselemente zwischen dem Tibiateil 20 und dem Femurteil 14, bekannt beispielsweise in Form von Vorsprüngen und entsprechenden Nocken, noch weitere Kondylen am Femurteil, also insbesondere keine dritte Kondyle, erforderlich.

Das Femurteil 14 und das Tibiateil 20 sind vorzugsweise aus einem Instrumentenstahl oder Titan oder einer Titanlegierung hergestellt, das Meniskusteil 16 bevorzugt aus einem abriebfesten Kunststoff, beispielsweise Polyethylen (PE).

## Patentansprüche

1. Kniegelenkendoprothese (10) mit einem Femurteil (14) und einem relativ zu und an diesem beweglich gelagerten Meniskusteil (16), welches Femurteil (14) eine mediale und eine laterale Kondyle (32, 34) umfasst, welche eine mediale und eine laterale Kondylenfläche (36, 38) aufweisen, welches Meniskusteil (16) eine mediale und eine laterale Gelenkfläche (50, 60) aufweist, an welchen die medialen und lateralen Kondylenflächen (36, 38) mindestens teilweise anliegen, wobei die mediale Kondyle (32) und die mediale Gelenkfläche (50) geformt sind zur Ausbildung eines Rotationsgelenks (56) mit einem Rotationsgelenkzentrum (52), wobei eine Abrollbewegungsführungseinrichtung (62) vorgesehen ist zum definierten Abrollen der lateralen Kondylenfläche (38) und der lateralen Gelenkfläche (60) aneinander längs einer in Abhängigkeit eines Flexionswinkels (68) zwischen Femurteil (14) und Meniskusteil (16) definierten, um das Rotationsgelenkzentrum (52) verlaufenden, gekrümmten Bahn (72), **dadurch gekennzeichnet, dass** das Rotationsgelenkzentrum (52) in Form eines relativ zum Femurteil (14) und zum Meniskusteil (16) längs einer in Abhängigkeit eines Flexionswinkels (68) zwischen Femurteil (14) und Meniskusteil (16) von anterior nach posterior verlaufenden Rotationsgelenkzentrumsbahn (54) wandernden Gelenkzentrums (52) ausgebildet ist.

2. Kniegelenkendoprothese nach Anspruch 1, **dadurch gekennzeichnet, dass** das Rotationsgelenkzentrum (52) in Form eines relativ zum Femurteil (14) und zum Meniskusteil (16) ortsfesten Gelenkzentrums (52) ausgebildet ist.

3. Kniegelenkendoprothese nach Anspruch 2, **dadurch gekennzeichnet, dass** das ortsfeste Gelenkzentrum (52) derart ausgebildet ist, dass es ausschließlich eine Gleitbewegung des Femurteils (14) und des Meniskusteils (16) relativ zueinander ermöglicht.

4. Kniegelenkendoprothese nach einem der voranstehenden Ansprüche, **dadurch gekennzeichnet, dass** die Rotationsgelenkzentrumsbahn (54) geradlinig verläuft oder vom Meniskusteil (16) in medialer Richtung weg weisend konvex gekrümmt ist.

5. Kniegelenkendoprothese nach einem der voranstehenden Ansprüche, **dadurch gekennzeichnet, dass** ein Krümmungsradius der medialen Gelenkfläche (50) größer ist als ein Krümmungsradius der medialen Kondylenfläche (36)
und/oder
dass das wandernde Gelenkzentrum (52) derart ausgebildet ist, dass es eine überlagerte Gleit-/Abrollbewegung des Femurteils (14) und des Meniskusteils (16) relativ zueinander ermöglicht.

6. Kniegelenkendoprothese nach einem der voranstehenden Ansprüche, **dadurch gekennzeichnet, dass** die Abrollbewegungsführungseinrichtung (62) miteinander zusammenwirkende erste und zweite Führungselemente (74, 76) umfasst, welche am Femurteil (14) und am Meniskusteil (16) ausgebildet sind zum Definieren eines in Abhängigkeit eines Flexionswinkels (68) zwischen Femurteil (14) und Meniskusteil (16) von anterior nach posterior und umgekehrt um das Rotationsgelenkzentrum (52) wandernden Kontaktflächenbereichs (92).

7. Kniegelenkendoprothese nach Anspruch 6, **dadurch gekennzeichnet, dass** das Femurteil (14) mindestens ein erstes und dass das Meniskusteil (16) mindestens ein zweites Führungselement (74, 76) aufweist, welche mindestens einen ersten und zweiten Führungselemente (74, 76) mindestens teilweise aneinander anliegende erste und zweite Führungselementflächen (94, 96) definieren.

8. Kniegelenkendoprothese nach Anspruch 7, **dadurch gekennzeichnet, dass** die erste Führungselementfläche (94) mindestens einen in Richtung auf das Meniskusteil (16) weisenden konkaven Flächenbereich (98) aufweist.

9. Kniegelenkendoprothese nach Anspruch 8, **dadurch gekennzeichnet, dass** die erste Führungselementfläche (94) zwei oder mehr, durch jeweils einen konvexen Flächenbereich (100) getrennte konkave Flächenbereiche (98) aufweist.

10. Kniegelenkendoprothese nach einem der Ansprüche 7 bis 9, **dadurch gekennzeichnet, dass** die zweite Führungselementfläche (96) mindestens einen in Richtung auf das Femurteil (14) weisenden konvexen Flächenbereich (102) aufweist.

11. Kniegelenkendoprothese nach Anspruch 10, **dadurch gekennzeichnet, dass** die zweite Führungselementfläche (96) zwei oder mehr, durch jeweils einen konkaven Flächenbereich (104) getrennte konvexe Flächenbereiche (102) aufweist.

12. Kniegelenkendoprothese nach einem der voranstehenden Ansprüche, **dadurch gekennzeichnet, dass** die laterale Kondylenfläche (38) mindestens einen in Richtung auf das Meniskusteil (16) weisenden konkaven Flächenbereich (98) aufweist.

13. Kniegelenkendoprothese nach Anspruch 12, **dadurch gekennzeichnet, dass** die laterale Kondylenfläche (38) zwei oder mehr, durch jeweils einen konvexen Flächenbereich (100) getrennte konkave Flächenbereiche (98) aufweist.

14. Kniegelenkendoprothese nach einem der voranstehenden Ansprüche, **dadurch gekennzeichnet, dass** die laterale Gelenkfläche (60) mindestens einen in Richtung auf das Femurteil (14) weisenden konvexen Flächenbereich (102) aufweist.

15. Kniegelenkendoprothese nach Anspruch 14, **dadurch gekennzeichnet, dass** die laterale Gelenkfläche (60) zwei oder mehr, durch jeweils einen konkaven Flächenbereich (104) getrennte konvexe Flächenbereiche (102) aufweist.

## Claims

1. Knee joint endoprosthesis (10) comprising a femoral component (14) and a meniscal component (16) mounted for movement relative to and on the femoral component, which femoral component (14) comprises a medial and a lateral condyle (32, 34) having a medial and a lateral condylar surface (36, 38), which meniscal component (16) comprises a medial and a lateral joint surface (50, 60) on which the medial and lateral condylar surfaces (36, 38) bear at least partially, the medial condyle (32) and the medial joint surface (50) being shaped so as to form a rotary joint (56) with a rotary joint center (52), wherein a rolling motion guiding device (62) is provided for defined rolling of the lateral condylar surface (38) and the lateral joint surface (60) on each other along a curved path (72) which is defined in dependence upon an angle of flexion (68) between femoral component (14) and meniscal component (16) and extends around the rotary joint center (52), **characterized in that** the rotary joint center (52) is in the form of a joint center (52) moving relative to the femoral component (14) and to the meniscal component (16) along a rotary joint center path (54) extending from anterior to posterior in dependence upon an angle of flexion (68) between femoral component (14) and meniscal component (16).

2. Knee joint endoprosthesis in accordance with claim 1, **characterized in that** the rotary joint center (52) is in the form of a joint center (52) which is fixed relative to the femoral component (14) and to the meniscal component (16).

3. Knee joint endoprosthesis in accordance with claim 2, **characterized in that** the fixed joint center (52) is configured so as to exclusively enable a sliding motion of the femoral component (14) and the meniscal component (16) relative to each other.

4. Knee joint endoprosthesis in accordance with any one of the preceding claims, **characterized in that** the rotary joint center path (54) extends in a straight line or is convexly curved facing away from the meniscal component (16) in medial direction.

5. Knee joint endoprosthesis in accordance with any one of the preceding claims, **characterized in that** a radius of curvature of the medial joint surface (50) is larger than a radius of curvature of the medial condylar surface (36),
and/or
**in that** the moving joint center (52) is configured so as to enable a superimposed sliding/rolling motion of the femoral component (14) and the meniscal component (16) relative to each other.

6. Knee joint endoprosthesis in accordance with any one of the preceding claims, **characterized in that** the rolling motion guiding device (62) comprises first and second guiding elements (74, 76) interacting with each other, which are formed on the femoral component (14) and on the meniscal component (16) to define a contact surface area (92) moving from anterior to posterior and vice versa around the rotary joint center (52) in dependence upon an angle of flexion (68) between femoral component (14) and meniscal component (16).

7. Knee joint endoprosthesis in accordance with claim 6, **characterized in that** the femoral component (14) comprises at least one first, and **in that** the meniscal component (16) comprises at least one second guiding element (74, 76), which at least one first and second guiding elements (74, 76) define first and second guiding element surfaces (94, 96) bearing at least partially on each other.

8. Knee joint endoprosthesis in accordance with claim 7, **characterized in that** the first guiding element surface (94) comprises at least one concave surface area (98) facing in the direction towards the meniscal component (16).

9. Knee joint endoprosthesis in accordance with claim 8, **characterized in that** the first guiding element surface (94) comprises two or more concave surface areas (98) which are separated in each case by a convex surface area (100).

10. Knee joint endoprosthesis in accordance with any one of claims 7 to 9, **characterized in that** the second guiding element surface (96) comprises at least one convex surface area (102) facing in the direction towards the femoral component (14).

11. Knee joint endoprosthesis in accordance with claim 10, **characterized in that** the second guiding element surface (96) comprises two or more convex surface areas (102) which are separated in each case by a concave surface area (104).

12. Knee joint endoprosthesis in accordance with any one of the preceding claims, **characterized in that** the lateral condylar surface (38) comprises at least one concave surface area (98) facing in the direction towards the meniscal component (16).

13. Knee joint endoprosthesis in accordance with claim 12, **characterized in that** the lateral condylar surface (38) comprises two or more concave surface areas (98), which are separated in each case by a convex surface area (100).

14. Knee joint endoprosthesis in accordance with any one of the preceding claims, **characterized in that** the lateral joint surface (60) comprises at least one convex surface area (102) facing in the direction towards the femoral component (14).

15. Knee joint endoprosthesis in accordance with claim 14, **characterized in that** the lateral joint surface (60) comprises two or more convex surface areas (102) which are separated in each case by a concave surface area (104).

## Revendications

1. Endoprothèse d'articulation du genou (10) comprenant une pièce de fémur (14) et une pièce de ménisque (16) montée de manière mobile par rapport à la pièce de fémur et sur celle-ci, ladite pièce de fémur (14) comportant un condyle médial et un condyle latéral (32, 34), qui présentent une surface condylienne médiale et une surface condylienne latérale (36, 38), ladite pièce de ménisque (16) présentant une surface articulaire médiale et une surface articulaire latérale (50, 60), sur lesquelles s'appuient au moins partiellement les surfaces condyliennes médiale et latérale (36, 38), endoprothèse
dans laquelle le condyle médial (32) et la surface articulaire médiale (50) sont formés pour réaliser une articulation de rotation (56) avec un centre d'articulation de rotation (52), et
dans laquelle il est prévu un dispositif de guidage de mouvement de roulement (62) pour assurer un roulement défini de la surface condylienne latérale (38) et de la surface articulaire latérale (60) l'une contre l'autre, le long d'une trajectoire courbe (72) définie en fonction d'un angle de flexion (68) entre la pièce de fémur (14) et la pièce de ménisque (16) et s'étendant autour du centre d'articulation de rotation (52), **caractérisée en ce que** le centre d'articulation de rotation (52) est réalisé sous la forme d'un centre d'articulation (52) se déplaçant par rapport à la pièce de fémur (14) et à la pièce de ménisque (16) le long d'une trajectoire de centre d'articulation de rotation (54) s'étendant d'une région antérieure à une région postérieure, en fonction d'un angle de flexion (68) entre la pièce de fémur (14) et la pièce de ménisque (16).

2. Endoprothèse d'articulation du genou selon la revendication 1, **caractérisée en ce que** le centre d'articulation de rotation (52) est réalisé sous la forme d'un centre d'articulation (52) en position fixe par rapport à la pièce de fémur (14) et à la pièce de ménisque (16).

3. Endoprothèse d'articulation du genou selon la revendication 2, **caractérisée en ce que** le centre d'articulation (52) en position fixe est configuré de manière à permettre exclusivement un mouvement de glissement de la pièce de fémur (14) et de la pièce de ménisque (16) l'une par rapport à l'autre.

4. Endoprothèse d'articulation du genou selon l'une des revendications précédentes, **caractérisée en ce que** la trajectoire de centre d'articulation de rotation (54) s'étend de manière rectiligne ou bien est courbée de manière convexe en s'éloignant de la pièce de ménisque (16) dans la direction médiale.

5. Endoprothèse d'articulation du genou selon l'une des revendications précédentes, **caractérisée en ce qu'**un rayon de courbure de la surface articulaire médiale (50) est supérieur à un rayon de courbure de la surface condylienne médiale (36),
et/ou
**en ce que** le centre d'articulation (52) est configuré de manière à permettre un mouvement superposé de glissement et roulement de la pièce de fémur (14) et de la pièce de ménisque (16) l'une par rapport à l'autre.

6. Endoprothèse d'articulation du genou selon l'une des revendications précédentes, **caractérisée en ce que** le dispositif de guidage de mouvement de roulement (62) comprend des premier et deuxième éléments de guidage (74, 76) interagissant mutuellement, qui sont réalisés sur la pièce de fémur (14) et sur la pièce de ménisque (16) pour définir une zone de surface de contact (92) se déplaçant autour du centre d'articulation de rotation (52) d'une région antérieure à une région postérieure et inversement, en fonction d'un angle de flexion (68) entre la pièce de fémur (14) et la pièce de ménisque (16).

7. Endoprothèse d'articulation du genou selon la revendication 6, **caractérisée en ce que** la pièce de fémur (14) présente au moins un premier et la pièce de ménisque (16) au moins un deuxième élément de guidage (74, 76), lesdits au moins un premier et deuxième éléments de guidage (74, 76) définissant des première et deuxième surfaces d'élément de guidage (94, 96) s'appuyant au moins partiellement l'une sur l'autre.

8. Endoprothèse d'articulation du genou selon la revendication 7, **caractérisée en ce que** la première surface d'élément de guidage (94) présente au moins une zone de surface concave (98) dirigée vers la pièce de ménisque (16).

9. Endoprothèse d'articulation du genou selon la revendication 8, **caractérisée en ce que** la première surface d'élément de guidage (94) présente deux zones de surface concaves (98) ou davantage, séparées respectivement par une zone de surface convexe (100).

10. Endoprothèse d'articulation du genou selon l'une des revendications 7 à 9, **caractérisée en ce que** la deuxième surface d'élément de guidage (96) présente au moins une zone de surface convexe (102) dirigée vers la pièce de fémur (14).

11. Endoprothèse d'articulation du genou selon la revendication 10, **caractérisée en ce que** la deuxième surface d'élément de guidage (96) présente deux zones de surface convexes (102) ou davantage, séparées respectivement par une zone de surface concave (104).

12. Endoprothèse d'articulation du genou selon l'une des revendications précédentes, **caractérisée en ce que** la surface condylienne latérale (38) présente au moins une zone de surface concave (98) dirigée vers la pièce de ménisque (16).

13. Endoprothèse d'articulation du genou selon la revendication 12, **caractérisée en ce que** la surface condylienne latérale (38) présente deux zones de surface concaves (98) ou davantage, séparées respectivement par une zone de surface convexe (100).

14. Endoprothèse d'articulation du genou selon l'une des revendications précédentes, **caractérisée en ce que** la surface articulaire latérale (60) présente au moins une zone de surface convexe (102) dirigée vers la pièce de fémur (14).

15. Endoprothèse d'articulation du genou selon la revendication 14, **caractérisée en ce que** la surface articulaire latérale (60) présente deux zones de surface convexes (102) ou davantage, séparées respectivement par une zone de surface concave (104).
